Europäisches Patentamt

European Patent Office

Office européen des brevets

(19)

(11) EP 1 457 211 A1

(12) EUROPEAN PATENT APPLICATION
published in accordance with Art. 158(3) EPC

(43) Date of publication:
15.09.2004 Bulletin 2004/38

(51) Int Cl.7: **A61K 39/285**, A61K 39/21,
C12N 7/01, C12N 15/49,
C12P 21/00

(21) Application number: 01274763.0

(22) Date of filing: 20.11.2001

(86) International application number:
PCT/JP2001/010141

(87) International publication number:
WO 2003/043654 (30.05.2003 Gazette 2003/22)

(84) Designated Contracting States:
AT BE CH CY DE DK ES FI FR GB GR IE IT LI LU
MC NL PT SE TR

(71) Applicants:
• Japan Science and Technology Agency
Kawaguchi-shi, Saitama 332-0012 (JP)
• Japan as represented by Director General of
National Institute of Infectious Diseases
Tokyo 162 (JP)

(72) Inventors:
• HONDA, Mitsuo
Mitaka-shi, Tokyo 181-0013 (JP)
• MATSUO, Kazuhiro
Kawasaki-shi, Kanagawa (JP)

• OHSU, Takeaki
Kawasaki-shi, Kanagawa (JP)
• MIYAMURA, Tatsuo
Tokyo 168-0065 (JP)
• MATSUURA, Yoshiharu
Suita-shi, Osaka (JP)
• ISHII, Koji
Tokyo (JP)
• KATO, Kenzo
Yachiyo-shi, Chiba 276-0046 (JP)

(74) Representative: Owen, Deborah Jane
Frank B. Dehn & Co.
179 Queen Victoria Street
London EC4V 4EL (GB)

(54) **RECOMBINANT VACCINIA VIRUS VACCINE**

(57)   The invention provides a recombinant vaccinia virus strain DIs that possesses a polynucleotide encoding a foreign antigenic protein in the non-essential gene region of the chromosome DNA and expresses the antigenic protein; and provides a highly-safe, vaccinia virus vaccine containing the recombinant virus strain DIs as the active ingredient. The invention also provides a method of using the vaccinia virus strain DIs as a vector for protein expression.

EP 1 457 211 A1

## Description

## Technical Field

The invention of this application relates to a recombinant vaccinia virus vaccine. More precisely, the invention of this application relates to a virus vaccine for various infectious diseases of humans and animals, and to a recombinant vaccinia virus strain DIs that serves as the effective ingredient of the vaccine.

## Background Art

With the development and improvement of the recombination technology in these ten years and more, various studies of denaturing microorganisms such as viruses and bacteria through genetic recombination are now made for application of the resulting recombinants to vaccine vectors for prevention and treatment of various infectious diseases and cancers. For the studies, various microorganisms have been tried, including, for example, polio virus, influenza viruses, rhino virus, chickenpox virus, Salmonella bacteria, bovine tubercle bacillus-attenuated strain BCG, and Listeria monocytogenes, for which, however, vaccinia virus has the oldest history of all.

Vaccinia virus is utilized as smallpox vaccines, and has much contributed toward eradicating smallpox. This virus is a large-size DNA virus, belonging to Poxviridae, and, as its genome, it has a linear double-stranded DNA of about 190 kbp, coding for about 200 types of different proteins. In 1982, Panicali and Paoletti (Proc. Natl. Acad. Sci. USA 79:4927, 1982), and Mackett et al. (Proc. Natl. Acad. Sci. USA 79:7415, 1982) first reported the expression of a recombinant vaccinia virus with a thymidine kinase (TK) gene of herpes simplex virus inserted thereinto. After that, various types of different recombinant vaccinia viruses have been constructed and tried for application to immunological analysis and to vaccine production. However, it is said that, depending on the toxicity of the parent strains used for their recombination, some of such recombinant vaccinia viruses will often induce encephalitis in humans and their toxicity will be often strengthened through atavism. From the viewpoint of safety, therefore, it is difficult to use the conventional recombinant vaccinia viruses for clinical vaccines for human use, especially for those for human immunodeficiency disorders such as AIDS, etc.

On the other hand, a vaccinia virus strain DIs is highly attenuated, and it was isolated through subcultivation of a vaccinia virus strain Talien (DEI) in hen's egg embryos for developing safe smallpox vaccines (Nature 192:381, 1961). The This strain DIs is specifically characterized in that it grows and propagates in fetal avian primary culture cells (DEF) but little in any other mammal cells. In those days, this was tried for clinical application along with a strain LC16 that had been developed together with it, and 200 Japanese children were actually vaccinated with it. However, as lacking the ability to propagate in human cells, the strain DIs was unfavorable for smallpox vaccines because of its poor immune inducibility, and the other strain LC16 better than the strain DIs in point of immune induction was employed for smallpox vaccines. This is the history of the strain DIs.

Still other attenuated vaccinia viruses, MVA (vaccinia virus strain Ankara) (Zur Beurteilung der MVA-Stufenimpfung bei Pockenerstrimpfungen, Med. Diss. Munchen, 1976) and NYVAC (vaccinia virus strain New York) (Virology 188:217-232) were reported. In particular, it was suggested that MVA could serve as a smallpox vaccine in humans. At present, MVA is propagated in mammal-derived cells such as CV-1 or BHK-16, and the attenuated virus MVA is now used as a vector for constructing various recombinant vaccines. However, the above-mentioned, attenuated strain DIs differs from the other attenuated strains MVA and NYVAC in that the former DIs does not propagate at all in mammal cells and is therefore useless for human vaccination.

The inventors of this application had succeeded in inserting a foreign gene into the genome of this strain DIs, and had verified that the recombinant strain DIs is useful as a viral vector for animal cells. However, since the immune inducibility of the strain. DIs is poor as so mentioned hereinabove, it has been believed that, even if the strain DIs is so recombined with a foreign antigenic protein as to express the foreign antigenic protein, the resulting recombinant strain DIs will be ineffective for vaccines.

## Disclosure of Invention

As so mentioned hereinabove, the cytotoxicity of the vaccinia virus strain DIs is low, it is easy to believe that the strain will be better than any other strains in point of its safety in human applications. Despite of the situation, however, because the immune inducibility of the strain DIs is poor, no one has heretofore considered the use of the strain DIs for recombinant virus vaccines. Contrary to this, the inventors of the present application have succeeded in introducing a foreign gene into the vaccinia virus strain DIs and in expressing the foreign gene in the recombinant strain DIs, and, in animal experiments, the inventors have found the possibility of inducing the humoral and cellular immune reaction in the foreign gene product from the recombinant vaccinia virus strain DIs.

In addition, since the vaccinia virus strain DIs does not propagate in animal cells, it has heretofore been believed that the strain will be unsuitable for an expression vector for useful proteins. However, the inventors of this application have found that, owing to its low cytotoxicity, the vaccinia virus strain DIs enables its host cells to live for a long period of time and is therefore useful as a vector for mass-expression of proteins.

The invention of this application has been made on the basis of the novel findings of the inventors as above, and its object is to provide a recombinant vaccinia virus vaccine of high safety.

Another object of the invention is to provide the recombinant vaccinia virus strain DIs that serves as the active ingredient of the vaccine.

Still another object of the invention is to provide the recombinant vaccinia virus strain DIs that expresses a reporter molecule and is therefore useful for construction of other recombinant virus strains.

Still another object of the invention is to provide a method of using the vaccinia virus strain DIs for a vector.

To achieve the objects noted above, the invention of this application provides the following inventions (1) to (19):

(1) A vaccinia virus vaccine containing, as the active ingredient, a recombinant vaccinia virus strain DIs that possesses a polynucleotide encoding a foreign antigenic protein in the non-essential gene region of the chromosome DNA and expresses the antigenic protein.

(2) The vaccine of above (1), wherein the antigenic protein is one derived from a human immunodeficiency virus.

(3) The vaccine of above (2), wherein the antigenic protein of the human immunodeficiency virus is a gag gene product.

(4) The vaccine of above (1), wherein the antigenic protein is one derived from a simian immunodeficiency virus.

(5) The vaccine of above (4), wherein the antigenic protein of the simian immunodeficiency virus is a gag gene product.

(6). A recombinant vaccinia virus strain DIs that possesses a polynucleotide encoding a foreign antigenic protein in the non-essential gene region of the chromosome DNA and expresses the antigenic protein.

(7). The recombinant vaccinia virus strain DIs of above (6), wherein the antigenic protein is one derived from a human immunodeficiency virus.

(8) The recombinant vaccinia virus strain DIs of above (7), wherein the antigenic protein of the human immunodeficiency virus is a gag gene product.

(9) The recombinant vaccinia virus strain DIs of above (6), wherein the antigenic protein is one derived from a simian immunodeficiency virus.

(10) The recombinant vaccinia virus strain DIs of above (9), wherein the antigenic protein of the simian immunodeficiency virus is a gag gene product.

(11) The recombinant vaccinia virus strain DIs of any of above (6) to (10), wherein the non-essential gene region of the chromosome DNA is a thymidine kinase gene region.

(12) The recombinant vaccinia virus strain DIs of any of above (6) to (10), wherein the non-essential gene region of the chromosome DNA is a region deleted in the vaccinia virus strain DIs when compared with the parent strain.

( 13) A recombinant vaccinia virus strain DIs that possesses a polynucleotide encoding a reporter molecule in the non-essential region of the chromosome DNA and expresses the reporter molecule.

(14) The recombinant vaccinia virus strain DIs of above (13), wherein the reporter molecule is a green fluorescence protein or an E. coli β-galactosidase.

(15) The recombinant vaccinia virus strain DIs of above (13) or (14), wherein the non-essential gene region of the chromosome DNA is a thymidine kinase gene region.

(16) The recombinant vaccinia virus strain DIs of above (13) or (14), wherein the non-essential gene region of the chromosome DNA is a region deleted in the vaccinia virus strain DIs when compared with the parent strain.

(17) A method of protein expression, which comprises inserting a polynucleotide encoding a foreign protein, into the non-essential gene region of the chromosome DNA of a vaccinia virus strain DIs, followed by mass-expressing the exogenous protein in host cells.

(18) The method of protein expression as above (17), wherein the non-essential gene region of the chromosome DNA is a thymidine kinase gene region.

(19) The method of protein expression as above (17), wherein the non-essential gene region of the chromosome DNA is a region deleted in the vaccinia virus strain DIs when compared with the parent strain.

**Brief Description of Drawings**

Fig. 1 shows a strategy of transfer vector construction for introducing a HIV gag gene into the genome of a strain DIs.

Fig. 2 shows the data of Western blotting, indicating the expression of the gag antigen in the extract of CEF cells infected with an HIV gag gene-recombinant vaccinia strain DIs. Arrow: Gag protein, lane 1: rVV-DIs-lacZ and lane 2: rVV-DIs-gag B.

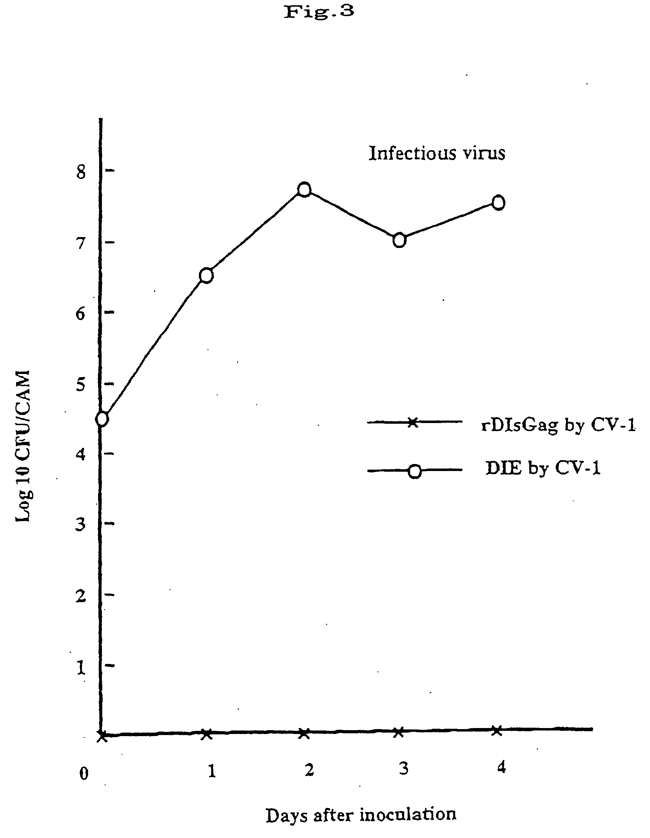

Fig. 3 shows the data of infection of CV-1 cells with the recombinant vaccinia virus strain DIs of this invention.

Fig. 4 shows the data of the ability of the recombinant vaccinia virus strain DIs of this invention to express its exogenous gene in different animal cells

Fig. 5 shows the data of CTL assay, indicating the HIV gag-specific CTL induction of rVV-DIs gagB in mice.

Fig. 6 shows the data of Western blotting, indicating the anti-HIV gag antibody production inducibility also in mice. Lane 1: normal control (HIV-1 infected human serum), lane 2: Normal MALB/c mouse serum, and lane 3: rVV-DIs gag B-immunized BALB/c mouse serum (12 weeks after immunization).

Fig. 7 shows the time-dependent change of CD4-positive lymphocytes in crab-eating monkeys immunized with the recombinant vaccinia virus strain DIs (rVV-DIs-SIV gag) of the invention (△, ○, ■) and in those not immunized with it (●), all the monkeys being infected with a pathogenic virus SHIV C21.

**The Best Mode for Carrying Out the Invention**

The vaccine of the inventions (1) to (5) is a highly-attenuated live vaccine that contains, as the active ingredient, the recombinant vaccinia virus strain DIs of the inventions (6) to (10). The recombinant vaccinia virus strain DIs of the invention (6) is a recombinant virus obtained through homologous recombination of the genome DNA of DIs with a transfer vector having a vaccinia virus-derived gene promoter sequence and having a polynucleotide that encodes an intended, foreign (that is, excluding vaccinia viruses) antigenic protein, linked downstream the promoter sequence. In the following description, the foreign antigenic protein will be referred to as "foreign polypeptide"; and the polynucleotide encoding it will be referred to as "foreign polynucleotide".
The promoter sequence to be inserted into the transfer vector may be any and every one capable of being recognized by the RNA polymerase which is encoded by the gene of a vaccinia virus itself. For it, for example, a 7.5 kD polypeptide gene promoter p7.5 that exists inside the inverted terminal repeats of the genome of a vaccinia virus (Stunnenberg, H. G., et al., Nucleic Acids Res., 16, 2431, 1988) may be used.
The foreign polynucleotide (e.g., cDNA fragment) may be any and every one that encodes an antigenic protein except vaccinia viruses; and the foreign polypeptide may be any and every one capable of inducing antigen-antibody reaction in an animal body. Concretely, herein directed thereto are a gag precursor protein p55, an env protein gp120 or gp160, a pol precursor protein, a nef protein and a tat protein of a human immunodeficiency virus (HIV) that causes human acquired immunodeficiency syndrome (AIDS); and a gag precursor protein of a simian immunodeficiency virus (SIV). In addition, also employable herein are other polynucleotides encoding other pathogens (other pathogenic viruses and bacteria) or for antigenic proteins of cancer cells.
Such foreign polynucleotides may be obtained, for example, by cleaving a plasmid clone of a genomic gene encoding an intended foreign polypeptide or its cDNA, or by amplifying through polymerase chain reaction (PCR) using primers having a suitable sequence and the plasmid clone as a template. If not cloned, the bacterial or animal genomic DNA having the gene is amplified through PCR for which is used a virus-infected animal cell-derived DNA or RNA as the template in case of viruses.

The thus-obtained foreign polynucleotide is ligated with the above-mentioned promoter in a correct reading frame, and the fused polynucleotide (this will be hereinafter referred to as "expression unit") is then inserted into a desired plasmid vector to construct a transfer vector.

Next, a method of homologous recombination for introducing the foreign polynucleotide into a vaccinia virus genome will be described. A vaccinia virus genome DNA is first extracted from virus particles which are purified through sucrose density gradient centrifugation (Joklik, W. K., Virology, 18, 9, 1962), using L-laurylsarucosine (Mackett, M. and Archard, L. C. J., Gen. Virol., 45, 683, 1979). In the thus-extracted genome DNA, the non-essential gene region that has no influence on the vaccinia virus replication must be specifically defined for the site for homologous recombination.

The first candidate for the non-essential gene region is a thymidine kinase (TK) gene region. In this case, inserting the TK gene region into a plasmid vector such as pUC18, and further inserting an expression unit at the unique restriction site in that region may construct a transfer vector. Using this vector, the foreign polynucleotide is inserted into the vaccinia virus genome through homologous recombination with the TK gene in the virus genome. After the homologous recombination, the recombinant virus is produced in the TK-lacking cell line, and incubated in the presence of bromodeoxyuridine to thereby select the recombinant virus not expressing TK.

The second candidate for the non-essential gene region is genomic gene-lacking region of DIs. Comparing the restriction enzyme cleavage patterns of the genomic DNA of DIs with that of the parent strain DIE may identify the defective region in the genome of DIs. This region is basically unnecessary for DIs replication. The DNA sequences before and after the defective region are amplified through PCR, and then inserted into a plasmid vector. An expression unit is further inserted into the plasmid to construct a transfer vector. Using the transfer vector, the DIs genomic DNA is subjected to homologous recombination, whereby the expression unit is inserted into the defective region of the DIs genomic DNA. In this method, however, the recombinant virus is difficult to select. To solve the problem, therefore, a polynucleotide encoding a reporter molecule is first inserted into the defective region to construct a recombinant virus. For the reporter molecule, for example, any of bacteria-derived coloring enzyme such as β-galactosidase, or a green fluorescence protein (GFP) or its derivatives (e.g., EGFP) may be used. For example, for the recombinant virus that expresses β-galactosidase, X-gal (5-bromo-4-chloro-3-indolyl-β-D-galactopyranoside) is added to the medium, by which the cells expressing β-galactosidase are selected based on their plaque color (blue). For GFP or its derivatives, the fluorescence-emitting plaques are selected through fluorescent microscopy. Into the reporter molecule-expressing virus, the intended foreign polynucleotide is inserted into the region of the reporter molecule through homologous recombination. In the process, the virus not expressing the reporter molecule is selected to thereby obtain the recombinant virus characterized by having the foreign polynucleotide inserted into the gene defective region through homologous recombination.

The thus-obtained recombinant virus is cloned and purified into a monoclone, and then confirmed for the presence or absence of the inserted gene therein through PCR. In Western blotting or enzyme-linked immunosorbent assay (ELISA), the monoclonal or polyclonal antibody against the foreign polypeptide inserted thereinto is tried in the ultrasonic extract of virus-infected CEF cells for confirming the expression of the foreign polypeptide in the infected cells.

The recombinant virus is grown in CEF cells, and then partially purified through sucrose density gradient centrifugation, and used in animal experiments. In animal experiments, mice and others such as rabbits and monkeys may be tried. For example, mice are intravenously, intramuscularly or intraperitoneally inoculated with the recombinant virus, and the antibody value to the foreign polypeptide in the serum may be measured at any intervals through Western blotting or ELISA to thereby determine the humoral immune response of the recombinant virus in mice. On the other hand, the cellular immune response of the recombinant virus in mice is determined as follows: First, mice are inoculated with the recombinant virus, then the lymphocytes in the blood collected from each mouse at any intervals are stimulated in vitro with the foreign polypeptide antigen, and the amount of the tritium thymidine intake into the lymphocytes is measured time-dependently. Secondly, foreign polypeptide antigen-specific cytotoxic T cells (CTL) in mice are identified as follows: After inoculated with the recombinant virus, the mice are grown for a predetermined period of time. Then, the spleen is taken out of each mouse, and mashed to obtain the spleen cells. The cells are stimulated in vitro with a synthetic peptide that corresponds to the CTL epitope in the foreign polypeptide, for a predetermined period of time. Thus stimulated, the cells serve as effector cells. On the other hand, the mouse cells of the same series are infected with the same recombinant vaccinia virus, or the mouse cells having been incubated along with a peptide antigen identified as the CTL epitope, and these cells are labeled with 51Cr to be target cells. The effector cells are reacted with the target cells in different ratios, and the radioactivity of 51Cr released in the culture supernatant is counted to thereby confirm the presence or absence of the CTL activity in the immunized mouse spleen cells.

The methods of the inventions (17) to (19) are for mass-expression of the foreign polynucleotide including useful proteins in the vaccinia virus strain DIs. Specifically, the vaccinia virus strain DIs accepts any type of polypeptide in a broad range for the foreign protein to be inserted thereinto, irrespective of the length and the structure of the foreign polynucleotide. In addition, the vaccinia virus strain DIs can be handled safely with no problem.

The recombinant vaccinia virus strain DIs is used as the expression vector in the methods, and it can be constructed in any desired manner using any desired foreign polynucleotide, like the recombinant virus strain DIs for vaccines mentioned above. The virus vector, strain DIs is transfected into any of CEF cells, mouse-derived p813 cells and fibroblasts in any ordinary method, and the resulting transfectant cells are incubated to thereby express a large amount of the protein that is encoded by the foreign polynucleotide inserted into the vector, for a long period of time. For example, in the experiments made by the inventors of this invention, the cells infected with the recombinant vaccinia virus strain DIs (concentration: 10 pfu) for 1 hour realized significant expression of the recombinant protein after incubation for 6 to 12 hours. In addition, the transfectant cells kept living for a long period of time, continuously expressing the protein.

## Examples

The invention of this application is described in detail and more concretely with reference to the following Examples, which, however, are not intended to restrict the scope of the invention.

## Example 1

## Construction of Recombinant Vaccinia Virus Strain DIs

In preparing a recombinant vaccinia virus from DIs, first investigated was the deletion site in the DIs genome. The parent strain DE1 and the DIs genome DNA were digested with HindIII, and the resulting fragments were compared through agarose gel electrophoresis. It was found that, of the 16 fragments, A to P (in the order of their size) of the parent strain genome DNA, the fragment N (1.5 kbp) and the fragment M (2.2 kbp) were deleted, the fragment C (25.1 kbp) and the fragment K (4.6 kbp) were shortened, and the 15.4 kbp region including almost all the 3'-side fragment C to the fragment K was lost in the DIs genome DNA. Accordingly, of the fragments deleted in the strain DIs, the fragment C and the fragment K, and the region (about 1.9 kbp) including the 5'-side of the fragment F (13.5 kbp) that is in contact with the adjacent fragment at the 3'-side of the fragment K were amplified through PCR and cloned in a PCR2.1 TA cloning vector (by Invitrogen). The PCR primers used are Vac H-C (oligonucleotide of SEQ ID NO. 1) and Vac H-F (oligonucleotide of SEQ ID NO. 2).

From the plasmid, the EcoRI fragment including the amplified region was cut out, and subcloned in the EcoRI site of pUC19 to obtain a vector pUC-DIs. The DIs fragment of the vector has one HindII site. In this site, an E. coli DNA fragment including the open reading frame of β-galactosidase gene (LacZ) was subcloned downstream the vaccinia virus latter-stage promoter p11 to give a transfer vector pUC-DIs-LacZ.

500 µl of a virus suspension containing about $10^5$ pfu (plaque-forming unit) of vaccinia virus DIs cells were inoculated into CEF cells (in a 6 cm-diameter laboratory dish), and the dish was lightly shaken four times at intervals of 15 minutes. After one hour, 2.5 ml of 1 % FBS/MEM medium was added thereto, and the cells were incubated for 6 hours under the condition of 37°C, 5 % $CO_2$. The medium was removed, and the cells were washed twice with PBS (phosphate buffered saline), then released in a trypsin-EDTA solution (by GIBCO), collected, and finally re-suspended in 400 µm of PBS. To the cell suspension, added was 20 µg of the transfer vector pUC-DIs-LacZ. In a 0.4 cm-cuvette, this was electroporated once at 250 V and 500 µFD (for this, used was BioRad's Gene Puisar II). The cells were then left at room temperature for 15 minutes or longer, and then spread in a laboratory dish and incubated therein for 4 days. The cells were released, collected along with the culture medium, then diluted 10-, 100- and 1000-fold, and thereafter inoculated into CEF cells for subcultivation in the same manner as above. After 4 days, when CPE was seen in the cells, the medium was removed, and an X-gal added agar medium (4-fold concentrated MEM medium 25 ml, distilled water 20.7 ml, 7.5 % $NaHCO_3$ 3 ml, 2.92 % L-glutamine 1.0 ml, 2 % X-gal 0.2 ml, 30 mg/ml kanamycin sulfate 0.1 ml, 2.4 % Agar Noble 50.0 ml) was added to the cells. After 4 hours, the blue-emitting CPE part of the agar was picked up, using a Pasteur pipette, and collected in 500 µl of the same medium as above. The thus-picked up agar was ultrasonically mashed, and then inoculated into CEF cells for subcultivation in the same manner as above. The same cycle as above was repeated to further clone the cells until the CPE in one laboratory dish with the cells therein was entirely in blue.

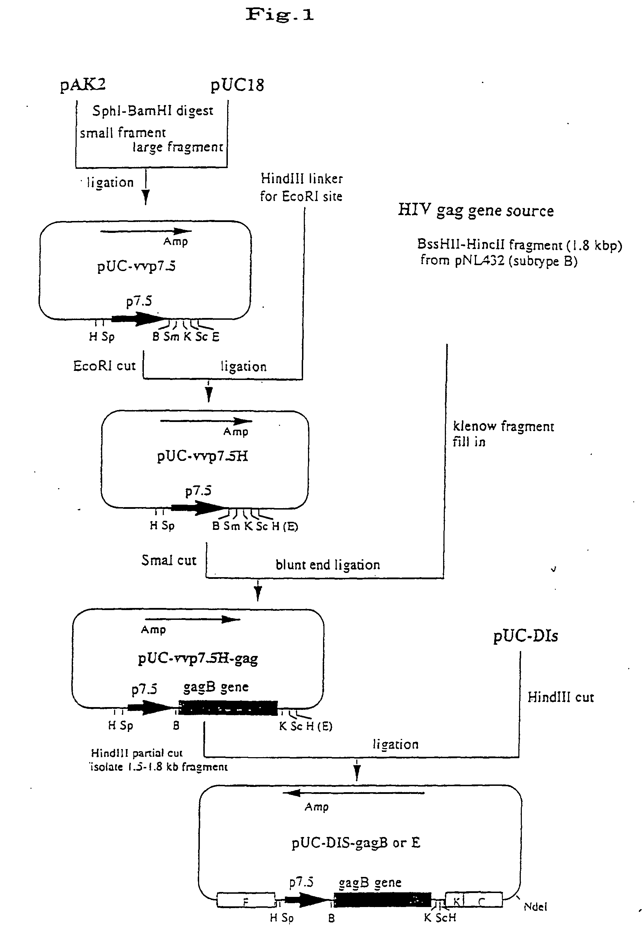

Next, in the HindIII site of the same transfer vector as above, inserted was an expression unit that had been constructed by linking the gag precursor protein gene of an HIV subtype B to the downstream of the promoter of a vaccinia virus p7.5. The outline of the transfer vector construction is shown in Fig. 1. Concretely, a subtype B HIV molecule clone pNL432 (Adachi, A., et al., J. Virol., 59, 284, 1986) was completely digested with restriction endonucleases BssHI and HincII, and the resulting fragment (about 1.8 kbp) was blunted with a Klenow fragment. This fragment was subcloned into the BamHI-SmaI site of a plasmid pUC-VVp7.5H that had been constructed by inserting the p7.5 promoter fragment cut out of a plasmid pAK2, into the PstI-XbaI site of pUC18, to thereby obtain pVV7.5-gagB. This plasmid was partially digested with HindIII, and its fragment (about 2.18 kbp) was separated and purified through 1 % agarose gel electrophoresis, and subcloned in the HindIII site of the transfer vector pUC-DIs to thereby obtain the intended vector pUC-DIs-gagB.

Similarly, CEF cells were infected with 500 µl of a virus suspension containing about $10^5$ pfu of cells of the β-galactosidase-expressing vaccinia virus strain DIs (rVV-DIs-LacZ) that had been prepared in the above, and the cells were released in a trypsin-EDTA solution, collected, and finally re-suspended in 400 µm of PBS. To the cell suspension, added was 20 µg of the transfer vector pUC-DIs-gagB. Then, this was electroporated under the same condition as above. The cells were then left at room temperature for 15 minutes or longer, and then spread in a laboratory dish and incubated therein for 4 days. The cells were released, collected along with the culture medium, then diluted 10-, 100- and 1000-fold, and thereafter inoculated into CEF cells for subcultivation in the same manner as above. After 4 days, when CPE was seen in the cells, the medium was removed, and an X-gal added agar medium was added to the cells. After 4 hours, the white CPE part of the agar not emitting in blue was collected in 500 µl of the same medium as above, using a Pasteur pipette. The thus-picked up agar was ultrasonically mashed, and then inoculated into CEF cells for subcultivation in the same manner as above. The same cycle as above was repeated to further clone the cells until the CPE in one laboratory dish with the cells therein did not emit in blue at all.

The HIV gag gene insertion into the thus-obtained recombinant virus was confirmed through PCR. For the primers for subtype B and E, used were ordinary SK38 (oligonucleotide of SEQ ID NO. 3: from 1551 to 1578) and SK 89 (oligonucleotide of SEQ ID NO. 4: from 1683 to 1665).

The white plaques formed indicate that 5 recombinant virus strains obtained herein all amplified the 114 bp DNA and had the HIV gag gene inserted therein.

Next, these recombinant viruses were tested for HIV gag antigen expression in virus-infected CEF cells.

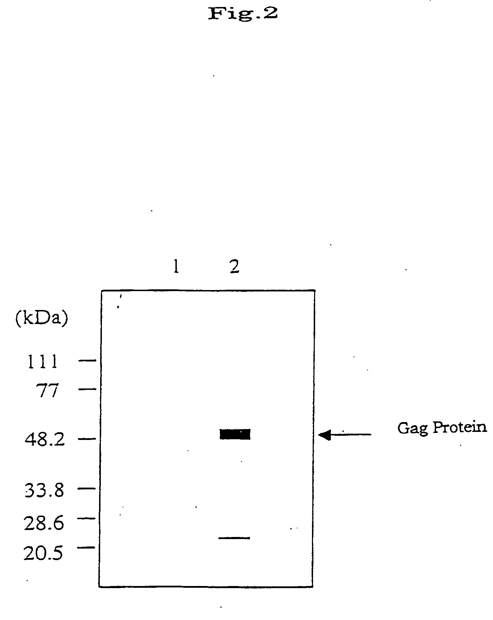

Fig. 2 shows the data of Western blotting of the infected cells. Concretely, the ultrasonic extract of the infected cells was separated through SDS-polyacrylamide gel electrophoresis, and assayed against an anti-gag monoclonal antibody (Matsuo, K., et al., J. Gen. Virol., 73, 2445, 1992). One of the 5 strains gave a specific band that might indicate the gag precursor p55 protein. In all the strains assayed, the insert protein lysates existed. The assay data indicate that the recombinant virus expressed the gag antigen. This strain was named rVV-DIs-gagB.

## Example 2

### Partial Purification of HIV-1 gag gene-expressing recombinant vaccinia virus strain DIs, and Determination of the quantity of virus

CEF cells were infected with rVV-DIs-gagB (in five laboratory dishes of 10 cm in diameter), and incubated in 10 ml of a 1 % PBS/MEM medium at 37°C in the presence of 5 % $CO_2$. After 2 to 4 days, the medium was removed when CPE was seen in the dishes. Then, the cells were disrupted in 15 ml of 10 mM Tris-HCl (pH 8.0), and then physically released and collected, and the virus having adhered to the cell debris was ultrasonically isolated. This was then centrifuged for 20 minutes at 3000 rpm, and the supernatant was further centrifuged for 90 minutes at 13000 rpm. The resulting mass of viruses was lysed in 8 ml of 10 mM Tris-HCl buffer capable of precipitating the viruses, to which was gently added 3 ml of 36 % sucrose. This was again centrifuged for 90 minutes at 13000 rpm. The resulting pellets were dissolved in 1 ml of 10 mM Tris-HCl buffer to prepare a partially-purified virus suspension. The virus suspension was stepwise diluted, and the quantity of viruses in the CEF cells was measured. It was $10^{10}$ pfu/ml.

## Example 3

### Investigation of Cell Propagation of recombinant vaccinia virus strain DIs

Different types of mammal-derived cells were incubated in a 48-well plate in a mode of monolayer culture, and infected with $10^5$ pfu of the recombinant virus strain rVV-DIs-LacZ that had been constructed in Example 1. After kept at 37°C for 1 hour, the cells of each type were separately washed with PBS, and further incubated in a fresh medium at 37°C. 0 hour and 48 hours after the absorption, the cells were collected, frozen and thawed. These were then ultrasonically processed, and the quantity of the viruses at 48 hours was divided by that at 0 hour to thereby determine the degree of virus propagation. The data are given in Table 1 below. In Table 1, Hela is a human-derived cell strain; CV-1 is an African green monkey-derived cell strain; RK is a rabbit kidney-derived cell strain; and CHO is a Chinese hamster ovary cell strain. As is obvious from Table 1, the parent strain (DEI) most actively propagated in CEF cells, and propagated in some degree in the other mammal cells except CHO. Contrary to this, however, the recombinant virus strain DIs did not propagate at all in the mammal cells except CEF cells.

Table 1

|  | HeLa | CV-1 | RK13 | CHO | CEF |
|---|---|---|---|---|---|
| Parent Strain | 7890 | 1578 | 842 | <1 | 9474 |
| rDIs | <1 | <1 | <1 | N.D. | 1600 |

Next, 2.0 moi of the recombinant vaccinia virus rVV-DIs-gagB constructed in Example 1 was incubated at 37°C for 1 hour along with CV-1 cells, and then cultured for 2 days. On the other hand, 0.1 moi of the parent strain (DEI) was also infected with CV-1 cells in the same manner, and then cultured for 4 days. After thus cultured, the cells of each type were separately recovered in 0.125 % trypsin-EDTA to prepare cell lysates. Each cell lysate was applied to 10-day-old eggs, in which the infectivity potency of each virus was determined.

The data are shown in Table 3. As in this, the parent vaccinia virus strain DEI infected the CV-1 cells, but the recombinant virus strain DIs of this invention did not at all.

## Example 4

### Investigation of Exogenous Gene Expression Potency of recombinant vaccinia virus strain DIs

A recombinant strain DIs capable of expressing a bacteriophage T7 polymerase was constructed in the same manner as in Example 1. Based on the method described in references, Virology 231;192-200, 1997, Virology 250: 140-150, 1998, the T7 polymerase activity (degree of expression) of the recombinant strain DIs was measured, using reporter genes pT7Luc and pT7EMCLuc. Concretely, different types of mammal-derived cells were incubated in a 48-well plate in a mode of monolayer culture, and infected with 2 moi of any of the strain DIs or the recombinant vaccinia virus rDIs-T7pol. For control, the cells were infected with 20 moi of a T7 polymerase-expressing adenovirus rAdexCAT7. After 24 hours, the cells of each type were transfected with 1 μg of a reporter plasmid pAcTU7Luc or pAcT7EMCLuc. The luciferase activity of the cells was measured in an assay kit, Pica Gene (by Tokyo Ink). 24 hours after thus transfected, the cells were collected, washed twice with PBS, and lysed in 100 μl of a cytolytic buffer LUC/PGC-50 (by Tokyo Ink). Next, 20 μl of each transparent cell lysate was incubated along with 100 μl of a reactive solution (containing 137 mM NaCl, 2.7 mM KCl, 4.3 mM $Na_2HPO_4$, 1.4 mM $KH_2PO_4$, 20 mM Tricine, 1.07 mM $(MgCO_3)_4Mg(OH)_2 \cdot 5H_2O$, 2.67 mM $MgSO_4$, 0.1mM EDTA, 33.3 mM DTT, 270 μM Coenzyme A, 470 μM luciferin, and 530 μM ATP). The relative luminosity unit (RLU) of each reaction liquid was measured with a luminometer (by Berthold).

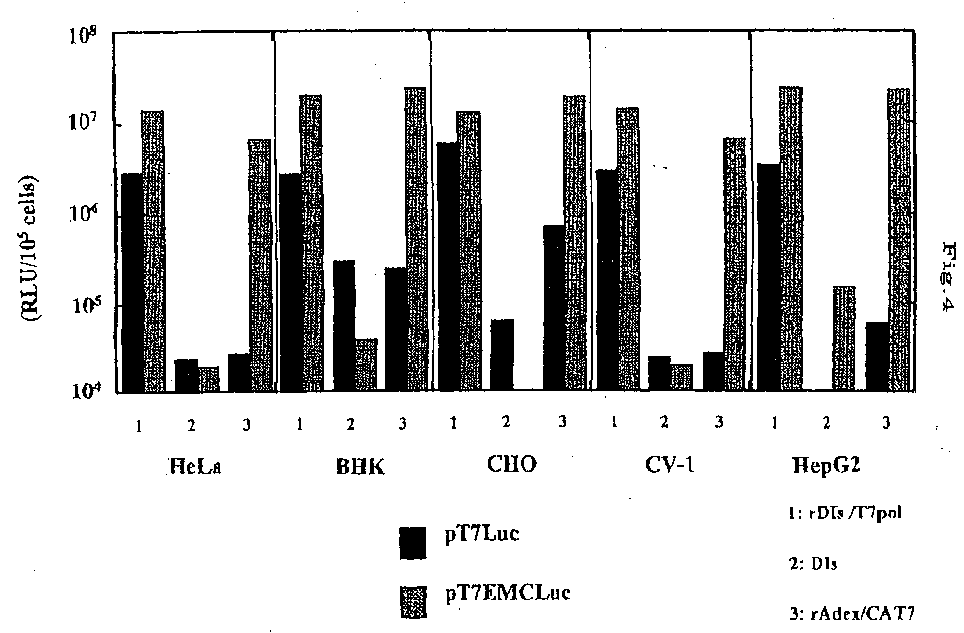

The data are shown in Fig. 4. In Fig. 4, BHK is a hamster baby cell strain; and HepG2 is a human-derived cell strain. As in Example 3, the recombinant vaccinia virus rDIs-T7pol has no propagation potency in the mammal-derived cells, but expressed the exogenous gene in the cells to the same degree as that of adenovirus. The results confirm that the recombinant vaccinia virus strain DIs of this invention, when used as a vaccine ingredient, does not so much propagate to an unnecessary degree, but expresses the intended antigenic protein necessary for antibody formation. The excellent characteristic of the recombinant vaccinia virus strain DIs of this invention supports the favorable applicability of the strain to vaccinia virus vaccines especially for patients suffering from immunodeficiency such as AIDS.

## Example 5

### Analysis for cellular immune induction against HIV-1 gag antigen in mice infected with recombinant vaccinia virus strain DIs that expresses HIV-1 gag gene

BALB/c mice were intravenously inoculated with $10^7$ pfu/mouse of rVV-DIs-gagB. At regular intervals of 2, 6, 8 and 12 weeks, the spleen was taken out of each mouse. Each spleen was mashed along with PBS in a cell strainer having a pore size of 70 μm (by Becton Dickinson), the resulting spleen cells were transferred into a 15-ml centrifugal tube and centrifuged. 5 ml of an ACK buffer (aqueous solution of 1.0 mM potassium bicarbonate, 0.15 M ammonium chloride and 0.1 mM of EDTA·2Na) was added to the resulting cell pellets, and mildly stirred to hemolyse the cells. 10 ml of PBS was added thereto and centrifuged, and the resulting pellets were washed twice with 10 ml of PBS. These were suspended in 5 ml of a 10 % FBS/RPMI1640 medium (by Nikken Biomedical Laboratory) containing $5 \times 10^{-5}$ M 2-mercaptomethanol, and the number of the live cells therein was counted. The suspension was diluted with the same medium to have a cell concentration of $1 \times 10^7$ cells/ml, to which was added an aqueous solution of two gag epitope peptides (concentration: 1 mg/ml) to have a final concentration of 10 μg/ml. Then, this was incubated at 37°C for 5 days. The amino acid sequences of the peptides (gag B253-277 and gag B287-309) are Sequence Numbers 5 and 6, respectively.

After 5 days, the number of the live cells in the suspension was again counted, and the cell concentration of the suspension was controlled to be $1 \times 10^7$ cells/ml with 10 % FBS/RPMI1640 added thereto. Thus processed, the cells are effector cells.

On the other hand, M 12.4.5 mouse cells (H2d haplo-type) were incubated in 10 % FBS/RPMI1640, and after reached 80 % confluence, these were washed once with PBS, then released in a trypsin-EDTA solution, and collected. These were suspended in 10 ml of 10 % FBS/RPMI1640, and the number of the live cells in the suspension was counted. Through centrifugation, $1 \times 10^7$ cells were collected, and suspended in 100 µm of the same medium. A solution of the two peptides as above was added to the suspension to have a final concentration of 50 µg/ml. For control, the peptide solution was not added to the cell suspension. Then, the cells were incubated at 37°C for 3 hours. Next, 100 µl (100 µCi) of an aqueous solution of 51Cr-labeled sodium chromate (by New England Biolab) was added to each cell suspension, and the cells were further incubated at 37°C in 5 % $CO_2$ for 1 hour and 30 minutes. Then, the cells were washed three times with 10 % FBS/RPMI1640, and then diluted with the same medium to have a cell concentration of $1 \times 10^5$ cells/ml. The cells are target cells.

The effector cells and the target cells were mixed in different ratios, 100/1, 50/1, 25/1 and 12.5/1 in a 96-well plate (total volume: 200 µl/well), and incubated at 37°C in 5 % $CO_2$ for 4 hours. To measure the spontaneous Cr emission and the maximum Cr emission, 100 µl of 10 % FBS/RPMI1640 or 100 µl of aqueous 1 % Triton×100 was added to 100 µl of the target cell suspension, and the cells were incubated under the same condition at 37°C in 5 % $CO_2$ for 4 hours. After 4 hours, the cells were deposited through centrifugation of the 96-well plate, and 20 µl of the supernatant was transferred into a luma plate (by Packard), left overnight as such to dry it in air. Using a gamma-ray counter (by Packard), the radioactivity of the dried supernatant was measured, and the CTL activity (%) of each sample was calculated according to the following formula:

$$\text{CTL activity (\%)}$$

$$= (\text{data of target cells - data of spontaneous emission/data of maximum}$$

$$\text{emission - data of spontaneous emission}) \times 100.$$

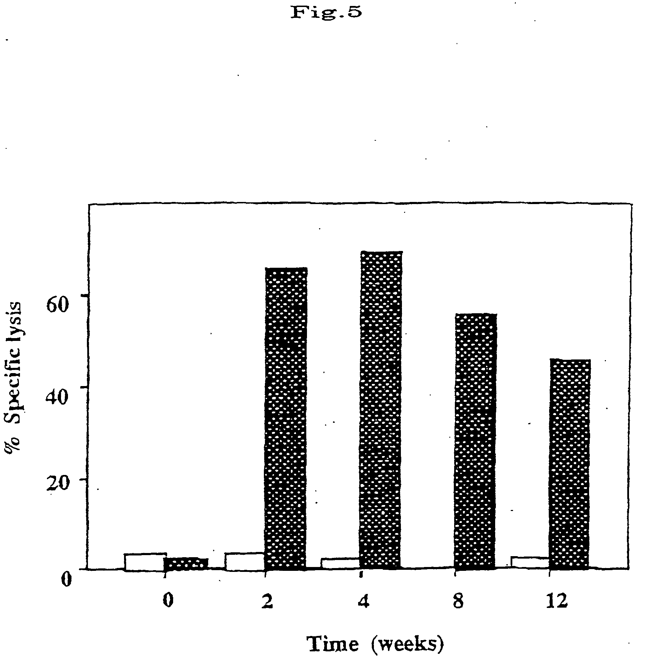

The results are shown in Fig. 5. On two weeks after the immunization, the CTL activity was not clear; but on 6 weeks, the anti-gag CTL activity was detected in one of two, and on 8 weeks, the activity was detected in two of two. Even on 12 weeks, the CTL activity in these was kept as such. These results confirm that the rVV-DIs-gagB keeps the CTL induction potency in mice.

### Example 6

**Analysis for induction of anti-HIV-1 gag antibody production in mice infected with recombinant vaccinia virus strain DIs that expresses HIV-1 gag gene**

As in Example 5, the rVV-DIs-gagB-immunized mouse blood was collected on 2, 6, 8 and 12 weeks after the immunization to prepare serum samples. Of those, 20 µl of the 12-weeks immunized serum was tested for its reactivity, using an HIV antigen-blotted nitrocellulose membrane filter (Genelabs Diagnostics' Western blotting kit).

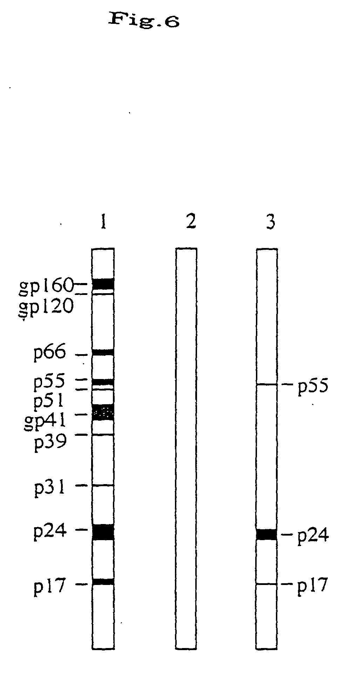

The results are shown in Fig. 6. The samples of the immunized group gave p55, p24 and p1 bands, but those of the non-immunized group did not. This supports the induction of anti-gag antibody production in the immunized mice.

### Example 7

**Construction of recombinant vaccinia virus strain DIs that expresses GFP derivative**

A plasmid pEGFP-1 (BY Clontach) was digested with EcoRI, and blunted through treatment with a Klenow fragment. The blunt DNA was further digested with BamHI, and the resulting fragment of about 700 bp was subcloned in the BamHI-SmaI site of pUC-VVp7.5H, like in Example 1. Next, this was cleaved with HindIII to obtain a fragment containing the region of p7.5 promoter and EGFP open reading frame. This was introduced into the HindIII site of pUC-DIs to construct a transfer vector. In the same manner as in Example 1, this plasmid was electroporated into CEF cells that had been infected with a β-galactosidase-expressing recombinant vaccinia virus strain DIs. With that, the cells were incubated on an X-gal agar medium, on which the cells giving white plaques were selected. These are of a recombinant virus strain. The virus-infected CEF cells were found to give green fluorescence when observed with a fluorescent microscope. This confirmed that the cells expressed the protein EGFP.

**Example 8**

**Construction of recombinant vaccinia virus strain DIs that expresses SIV gag gene**

The genetic clone pKS460 of a simian immunodeficiency virus SIV was digested with restriction endonucleases NdeI and XhoI, and blunted through treatment with a Klenow fragment. The resulting fragment of about 1500 bp was subcloned in the BamHI-SmaI site of pUC-VVp7.5H, like in Example 1. Next, this was cleaved with HindIII to obtain a fragment containing the region of p7.5 promoter and EGFP open reading frame. This was introduced into the HindIII site of pUC-DIs to construct a transfer vector. In the same manner as in Example 1, this plasmid was electroporated into CEF cells that had been infected with a β-galactosidase-expressing recombinant vaccinia virus strain DIs. With that, the cells were incubated on an X-gal agar medium, on which the cells giving white plaques were selected. These are of a recombinant virus strain rVV-DIs-SIV gag.

**Example 9**

**Evaluation of Immune Induction and Immune Protection in monkeys inoculated with recombinant vaccinia virus strain DIs that expresses SIV gag gene**

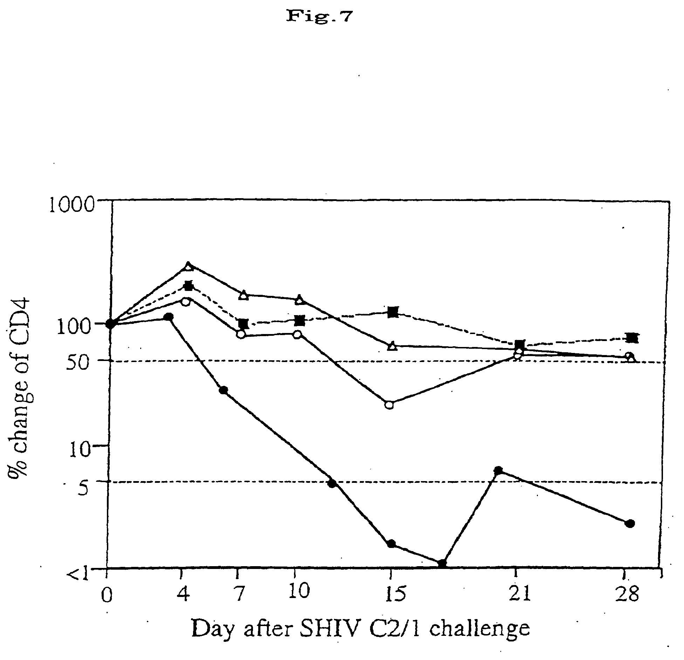

The recombinant virus rVV-DIs-SIV gag that had been constructed in Example 8 was intravenously administered to crab-eating monkeys twice at an interval of 45 days ($10^6$ pfu/monkey), and the immune induction in the thus-immunized monkeys was evaluated through a test for antibody production in the monkeys, a test for lymphocyte propagation therein, an ELISPOT assay and an intracellular cytokine production assay. In those tests and assays, all the rVV-DIs-SIV gag-immunized, crab-eating monkeys showed the induction of anti-gag antibody, gag-specific helper, and CTL activity. On the other hand, no abnormal observations were found in all the immunized monkeys.
30 days after the immunization with rVV-DIS-SIV gag, the monkeys were infected with a pathogenic chimera virus SHIV C21 10TCID, and the number of the CD4-positive lymphocytes in the peripheral blood collected from each monkey was counted. The results are shown in Fig. 7. As in this, the number of the CD4-positive lymphocytes significantly decreased in the monkeys of the control group within 2 weeks after the pathogenic virus challenge, while the reduction in the CD4-positive lymphocytes in the rVV-DIS-SIV gag-immunized monkeys was retarded by 70 to 95 %. In addition, it was further confirmed that, in the immunized monkeys, the number of viral RNA copies in plasma at set points was decreased to a degree of 1/50 to 1/100.
The results as above confirm that the recombinant virus vaccine of this invention has the potency of antigen-specific immune induction and immune protection in the primates.

**Industrial Applicability**

As described in detail hereinabove, the invention of this application provides an attenuated, recombinant vaccinia virus vaccine having high antigenicity against pathogens except vaccinia viruses, and provides a recombinant vaccinia virus strain DIs that serves as the active ingredient of the vaccine. The recombinant vaccinia virus strain DIs which the invention provides herein has the ability to induce both humoral and cellular immunity against the foreign polypeptide inserted thereinto. In addition, it is easy to change the foreign polypeptide in the recombinant strain with any other polypeptide through genetic recombination technology, the recombinant strain is useful for vectors for recombinant virus vaccines for various infectious diseases. Further, the recombinant virus does not propagate in ordinary animal cells but expresses the immunogenic antigen (having the potency of inducing cellular immunity and antibody production), and the virus cells are easy to incubate and can be produced at low costs. For these reasons, the recombinant strain of this invention can be used as an inexpensive substitute for antigens for boosts in the prime and boost method that is much used in the art for developing AIDS vaccines. Accordingly, the recombinant strain of this invention is extremely useful for antigens substitutable for pure recombinant proteins that require high production costs. Moreover, since the recombinant strain of this invention is highly safe to humans, it is useful for a recombinant vaccinia virus strain to be infected into target cells in CTL activity determination from the viewpoint of safe protection of test subjects.

**Claims**

1. A vaccinia virus vaccine containing, as the active ingredient, a recombinant vaccinia virus strain DIs that possesses a polynucleotide encoding a foreign antigenic protein in the non-essential gene region of the chromosome DNA and expresses the antigenic protein.

2. The vaccine of claim 1, wherein the antigenic protein is one derived from a human immunodeficiency virus.

3. The vaccine of claim 2, wherein the antigenic protein of the human immunodeficiency virus is a gag gene product.

4. The vaccine of claim 1, wherein the antigenic protein is one derived from a simian immunodeficiency virus.

5. The vaccine of claim 4, wherein the antigenic protein of the simian immunodeficiency virus is a gag gene product.

6. A recombinant vaccinia virus strain DIs that possesses a polynucleotide encoding a foreign antigenic protein in the non-essential gene region of the chromosome DNA and expresses the antigenic protein.

7. The recombinant vaccinia virus strain DIs of claim 6, wherein the antigenic protein is one derived from a human immunodeficiency virus.

8. The recombinant vaccinia virus strain DIs of claim 7, wherein the antigenic protein of the human immunodeficiency virus is a gag gene product.

9. The recombinant vaccinia virus strain DIs of claim 6, wherein the antigenic protein is one derived from a simian immunodeficiency virus.

10. The recombinant vaccinia virus strain DIs of claim 9, wherein the antigenic protein of the simian immunodeficiency virus is a gag gene product.

11. The recombinant vaccinia virus strain DIs of any of claims 6 to 10, wherein the non-essential gene region of the chromosome DNA is a thymidine kinase gene region.

12. The recombinant vaccinia virus strain DIs of any of claims 6 to 10, wherein the non-essential gene region of the chromosome DNA is a region deleted in the vaccinia virus strain DIs when compared with the parent strain.

13. A recombinant vaccinia virus strain DIs that possesses a polynucleotide encoding a reporter molecule in the non-essential region of the chromosome DNA and expresses the reporter molecule.

14. The recombinant vaccinia virus strain DIs of claim 13, wherein the reporter molecule is a green fluorescence protein or an E. coli β-galactosidase.

15. The recombinant vaccinia virus strain DIs of claim 13 or 14, wherein the non-essential gene region of the chromosome DNA is a thymidine kinase gene region.

16. The recombinant vaccinia virus strain DIs of claim 13 or 14, wherein the non-essential gene region of the chromosome DNA is a region deleted in the vaccinia virus strain DIs when compared with the parent strain.

17. A method of protein expression, which comprises inserting a polynucleotide encoding a foreign protein, into the non-essential gene region of the chromosome DNA of a vaccinia virus strain DIs, followed by mass-expressing the exogenous protein in host cells.

18. The method of protein expression as claimed in claim 17, wherein the non-essential gene region of the chromosome DNA is a thymidine kinase gene region.

19. The method of protein expression as claimed in claim 17, wherein the non-essential gene region of the chromosome DNA is a region deleted in the vaccinia virus strain DIs when compared with the parent strain.

# Fig.1

Fig.2

## Fig.3

EP 1 457 211 A1

$10^8$

$10^7$

(RLU/$10^5$ cells)

$10^6$

$10^5$

$10^4$

1   2   3   1   2   3   1   2   3   1   2   3   1   2   3

HeLa       BHK       CHO       CV-1       HepG2

Fig.4

■ pT7Luc

▨ pT7EMCLuc

1: rDIs /T7pol

2: DIs

3: rAdex/CAT7

Fig.5

Fig.6

Fig.7

**INTERNATIONAL SEARCH REPORT**

| International application No. |
|---|
| PCT/JP01/10141 |

**A. CLASSIFICATION OF SUBJECT MATTER**
Int.Cl⁷ A61K39/285, A61K39/21, C12N7/01, C12N15/49, C12P21/00

According to International Patent Classification (IPC) or to both national classification and IPC

**B. FIELDS SEARCHED**

Minimum documentation searched (classification system followed by classification symbols)
Int.Cl⁷ A61K39/285, A61K39/21, C12N7/01, C12N15/49, C12P21/00

Documentation searched other than minimum documentation to the extent that such documents are included in the fields searched

Electronic data base consulted during the international search (name of data base and, where practicable, search terms used)
JICST FILE(JOIS), MEDLINE(STN), BIOSIS(DIALOG)

**C. DOCUMENTS CONSIDERED TO BE RELEVANT**

| Category* | Citation of document, with indication, where appropriate, of the relevant passages | Relevant to claim No. |
|---|---|---|
| X | Takeaki OSU, et al., "AIDS Vaccine no Kaihatsu", Gene & Medicine, November, 1999, Vol.3, No.4, page 688 to 760 | 1-19 |
| Y | Ilnour OURMANOV, et al., Comparative Efficacy of Recombinant Modified Vaccinia Virus Ankara Expressing Simian Immunodeficiency Virus (SIV) Gag-Pol and/or Env in Macaques Challenged with Pathogenic SIV. Journal of Virology, March, 2000, Vol.74, No.6, pages 2740 to 2751 | 4,5,9,10 |
| Y | Aruna SETH, et al., Recombinant modified vaccinia cirus Ankara-simian immunodeficiency virus gag pol elicits cytotoxic T lymphocytes in rhesus monkeys detected by a major histocompatibility complex class I/peptide tetramer. Proc.Natl.Acad.Sci.USA, August 1998, Vol.95, pages 10112 to 10116 | 4,5,9,10 |

☐ Further documents are listed in the continuation of Box C.　　☐ See patent family annex.

| | |
|---|---|
| * Special categories of cited documents: | "T" later document published after the international filing date or priority date and not in conflict with the application but cited to understand the principle or theory underlying the invention |
| "A" document defining the general state of the art which is not considered to be of particular relevance | |
| "E" earlier document but published on or after the international filing date | "X" document of particular relevance; the claimed invention cannot be considered novel or cannot be considered to involve an inventive step when the document is taken alone |
| "L" document which may throw doubts on priority claim(s) or which is cited to establish the publication date of another citation or other special reason (as specified) | "Y" document of particular relevance; the claimed invention cannot be considered to involve an inventive step when the document is combined with one or more other such documents, such combination being obvious to a person skilled in the art |
| "O" document referring to an oral disclosure, use, exhibition or other means | |
| "P" document published prior to the international filing date but later than the priority date claimed | "&" document member of the same patent family |

| Date of the actual completion of the international search | Date of mailing of the international search report |
|---|---|
| 28 February, 2002 (28.02.02) | 12 March, 2002 (12.03.02) |

| Name and mailing address of the ISA/ | Authorized officer |
|---|---|
| Japanese Patent Office | |
| Facsimile No. | Telephone No. |

Form PCT/ISA/210 (second sheet) (July 1998)

SEQUENCE LISTING

<110> Japan Science and Technology Corporation

<120> Recombinant vaccinia virus vaccine

<130> 01-F-057PCT

<160> 6

<210> 1
<211> 30
<212> DNA
<213> Artificial sequence

<220>
<223> Artificial sequence: Synthesized oligonucleotide

<400> 1
ATAATGTAGC TCCTTCATCA ATCATACATT                              30

<210> 2
<211> 30
<212> DNA
<213> Artificial sequence

<220>
<223> Artificial sequence: Synthesized oligonucleotide

<400> 2

AGGAGGTGGT GTAATAGACG AAGATTATAG                                    30

<210> 3

<211> 28

<212> DNA

<213> Artificial sequence

<220>

<223> Artificial sequence: Synthesized oligonucleotide

<400> 3

ATAATCCACC TATCCCAGTA GGAGAAAT                                      28

<210> 4

<211> 28

<212> DNA

<213> Artificial sequence

<220>

<223> Artificial sequence: Synthesized oligonucleotide

<400> 4

TTTGGTCCTT GTCTTATGTC CAGAATGC                                      28

<210> 5

<211> 25

<212> PRT

<213> Artificial sequence

<220>

<223> Artificial sequence: Synthesized oligopeptide

<400> 5

Asn Pro Pro Ile Pro Val Gly Glu Ile Tyr Lys Arg Trp Ile Ile Leu
1               5               10              15
Gly Leu Asn Lys Ile Val Arg Met Tyr
                20              25

<210> 6
<211> 23
<212> PRT
<213> Artificial sequence

<220>

<223> Artificial sequence: Synthesized oligopeptide

<400> 6

Gln Gly Pro Lys Glu Pro Phe Arg Asp Tyr Val Asp Arg Phe Tyr Lys
1               5               10              15
Thr Leu Arg Ala Glu Gln Ala
                20      23